Europäisches Patentamt

(19) European Patent Office

Office européen des brevets

(11) EP 0 491 983 B1

(12) **EUROPEAN PATENT SPECIFICATION**

(45) Date of publication and mention
of the grant of the patent:
**20.03.1996 Bulletin 1996/12**

(51) Int. Cl.⁶: **A61L 31/00**, **A61B 17/58**

(21) Application number: **90125452.4**

(22) Date of filing: **24.12.1990**

---

(54) **Biodegradable and resorbable molded article for surgical use**

Biologisch abbaubarer und resorbierbarer Formkörper für chirurgischen Gebrauch

Article moulé biodégradable et résorbable pour utilisation chirurgicale

(84) Designated Contracting States:
**CH DE FR GB LI NL**

(43) Date of publication of application:
**01.07.1992 Bulletin 1992/27**

(73) Proprietor: **TAKIRON CO. LTD.**
**Osaka-shi Osaka 541 (JP)**

(72) Inventors:
• **Shikinami, Yasuo,**
**c/o Takiron Co. Ltd.**
**Osaka-shi, Osaka (JP)**
• **Tsuta, Kaoru,**
**c/o Takiron Co. Ltd.**
**Osaka-shi, Osaka (JP)**
• **Boutani, Hidekuzu,**
**c/o Takiron Co. Ltd.**
**Osaka-shi, Osaka (JP)**

(74) Representative: **Hansen, Bernd, Dr. Dipl.-Chem. et al**
**Hoffmann, Eitle & Partner**
**Patent- und Rechtsanwälte,**
**Postfach 81 04 20**
**D-81904 München (DE)**

(56) References cited:
**EP-A- 0 108 635**      **EP-A- 0 260 222**
**EP-A- 0 316 992**      **EP-A- 0 349 656**
**WO-A-90/06088**       **GB-A- 2 141 435**
**US-A- 4 898 186**

• **MAKROMOL. CHEM., suppl. 5, 1981, pages 30-41;
M. VERT et al.: "Stereoregular bioresorbable
polyesters for orthopaedic surgery"**

**Description**

FIELD OF THE INVENTION

This invention relates to a biodegradable and resorbable molded article for fixing or retaining and reinforcing a damaged or fractured part of bone for osteosynthesis. More particularly, it relates to a surgical polylactic acid molded article for osteosynthesis whose strength, shape, size, and rate of degradation or deterioration are controlled so that the strength thereof may be retained while osteogenesis in the damaged or fractured bone gradually proceeds to restore strength to a level substantially sufficing for daily living and that the article may be biodegraded and resorbed into a living body by the time osteogenesis is substantially accomplished.

In the fields of orthopedic surgery and oral surgery, artificial high strength materials for osteosynthesis, such as plates and screws, are used. These artificial materials are required to function until a fracture heals and must be removed after the healing as soon as possible to prevent weakening of the bone.

Most of osteosynthetic plates which are widespread for clinical use are made of metals. Ceramic plates have recently been developed. However, these conventional materials have disadvantages such that their high elastic modulus rather reduces strength of surrounding bones, or the high elastic modulus is sometimes accompanied with brittleness, and metallic ions eluted from metallic materials do harm to a living body. It is therefore expected that a biodegradable and resorbable material could be a promising material for osteosynthesis which would eliminate not only the necessity of re-operation for removal but various adverse influences on a living body during its long-term existence in the body.

Under these circumstances, an osteosynthetic material comprising polylactic acid or a lactic acid-glycolic acid copolymer which is a biodegradable and resorbable material has been developed to date.

For example, M. Vert, F. Chabot, et al. proposed osteosynthetic plates comprising polylactic acid or a lactic acid-glycolic acid copolymer. According to their report, a molded article comprising 100% polylactic acid has a bending modulus in compression of 3.4 GPa (340 kg/mm$^2$) as described in <u>Macromol. Chem. Suppl.</u>, Vol. 5, pp. 30-41 (1981). D.C. Tunc reported an osteosynthetic plate comprising polylactic acid having a bending modulus in compression of 520 kg/mm$^2$ as described in <u>Transactions of 9th Annual Meeting of the Society for Biomaterials of USA</u>, Vol. 6, P. 47 (1983) and U.S. Patent 4,279,249.

More specifically, U.S. Patent 4,279,249 proposes a biodegradable and resorbable molded article which comprises a fiber or fabric of a polyglycolic acid and a polylactic acid. But the molecular weight of the polylactic acid is small. In addition, it is impossible to improve the physical properties by molecular orientation upon stretching, because it compounds two components each having different glass transition temperature. Then it is difficult to obtain the initial bending strength of above 2,000 kg/cm$^2$. Furthermore, in view of processability, when it is molded by processing (e.g., cutting) to obtain the form of screw, plate, rod, pin, etc., the obtained article shows scattering results of the strength and the decomposition speed because of localization of the reinforcement material. It is difficult to provide, in view of physical properties, reliable materials for osteosynthesis.

JP-A-59-97654 (the term "JP-A" as used herein means an "unexamined published Japanese patent application") corresponding to U.S. Patent 4,898,186 discloses a process for synthesizing polylactic acid as a resorbable material for bone fixation. The disclosed polylactic acid had a low tensile strength as about 580 kg/cm$^2$ and, also, there is no reference to a method for molding the polylactic acid.

Further, J.W. Leenslag, A.J. Pennings, et al. synthesized polylactic acid having a viscosity average molecular weight of about 1,000,000, reporting that an osteosynthetic plate produced from the high molecular-weight polylactic acid has a bending modulus in compression of 5 GPa (500 kg/cm$^2$), as described in <u>Biomaterials</u>, Vol. 8, p. 70 (1987). The disclosed polylactic acid, however, has difficulty in molding on account of its too high molecular weight.

Although a number of reports have thus been made on study for improving mechanical properties of polylactic acid type osteosynthetic materials, a material having sufficient strength for clinical use has not yet been developed.

The inventors of the present invention previously proposed a biodegradable and resorbable polylactic acid molded article for surgery which has the same or a slightly higher strength as compared with bones in a living body as described in JP-A-1-198553. However, when the proposed material is actually implanted in a living body, it turned out that a strength required and a duration of strength largely vary depending on the site of implantation. That is, strength values of polylactic acid, such as bending strength in compression, bending modulus in compression, and tensile strength, which are suited for a particular site of bones of a human body have not yet been specified, and proper shape and size of the molded article for exhibiting such specific strength have not yet been elucidated. Moreover, shape and size of the molded article which are capable of retaining sufficient strength for a required time have not yet been made clear.

EP-A-0 349 656 discloses surgical article material as used in the invention but no dimensions are given for the surgical articles except for test pieces which differ in size from the articles used according to the invention.

## SUMMARY OF THE INVENTION

An object of the present invention is to provide a biodegradable and resorbable molded article of polylactic acid for osteosynthesis or bone fixation (or retention), such as a screw, a rod, a plate, and a pin, which has the same or slightly higher mechanical strength as compared with a bone at a specific site in a living body, which has a shape and a size suitable for retaining its strength while being implanted in a living body for a period required for osteoanagenesis, and which has a size fitted to the site of operation, not greater than necessary, requiring no extra time for degradation and absorption.

The present invention relates to a biodegradable and resorbable molded article for surgery which comprises a polylactic acid molded and stretched article having a viscosity average molecular weight of from 200,000 to 500,000, a bending strength in compression of from $16.0 \times 10^2$ to $25.0 \times 10^2$ kg/cm$^2$, a bending modulus in compression of from $5.5 \times 10^2$ to $24.0 \times 10^2$ kg/mm$^2$, and a degree of crystallinity of from 10 to 60% as calculated from density, the thickest portion of said molded and stretched article being controlled between 1.0 mm and 4.5 mm so that said article is deteriorated in strength due to degradation in a living body within about 4 to 16 weeks and resorbed into a living body at least 50 wt% during from about 1.5 to 2.0 years.

Preferred embodiments of the polylactic acid molded article according to the present invention include (1) a screw-shaped molded article having a diameter of from 2.5 to 3.5 mm which is used in a shoulder joint for fixation of the greater tuberosity, osteoclastic fragments of the collum chirurgicum, and fragments of the glenoid, (2) a pin-shaped molded article having a diameter of from 1.0 to 2.5 mm which is used in an elbow joint for osteochondral facture and fixation of a bone graft for curing baseball elbow, (3) a screw-shaped molded article having a diameter of from 2.0 to 3.0 mm which is useful in an elbow joint for osteosynthesis of a fracture of the medial epicondyle of humerus or lateral epicondyle of humerus, (4) a pin-shaped molded article having a diameter of from 1.0 to 2.0 mm which is used in carpal bones and metacarpals for osteosynthesis of a fracture of phalax of finger in various sites, an intraarticular fracture, a fracture of distal radius, (5) a microscrew-shaped molded article having a diameter of from 1.5 to 2.5 mm which is used in a wrist joint and a phalax of finger for osteosynthesis of a fracture of a distal radius and a fracture of a phalax of finger, (6) a pin- or screw-shaped molded article having a diameter of from 2.5 to 3.5 mm which is used in the pelvis and the hip-joint for osteosynthesis of a fracture of the acetabulum and an avulsion fracture of the ilium, for fixation of a graft in hip-joint arthroplasty, and for osteosynthesis of a fracture of femoral head, (7) a pin- or screw-shaped molded article having a diameter of from 3.5 to 4.5 mm which is used in a knee joint for osteosynthesis of a fracture of the femoral condyle, (8) a pin- or screw-shaped molded article having a diameter of from 2.5 to 3.5 mm which is used in a knee joint for osteosynthesis of an intraarticular fracture, an osteochondritis dissecans, a fracture of the proximal tibia or for osteosynthesis in osteotomy, (9) a screw-shaped molded article having a diameter of from 2.5 to 3.5 mm which is used for osteosynthesis of a fracture in the medial, lateral or posterior malleolus of an ankle joint, (10) a screw-shaped molded article having a diameter of from 3.5 to 4.5 mm which is used for osteosynthesis of a fracture of the talus in an ankle joint, (11) a pin-shaped molded article having a diameter of from 2.0 to 4.0 mm which is used in an ankle joint for osteosynthesis of an osteochondritis dissecans and an osteochondral fracture, (12) a pin-shaped molded article having a diameter of from 1.5 to 2.5 mm which is used in toes in an operation of a hallux valaus or osteosynthesis of a fracture of a phalanx of toe, (13) a screw-shaped molded article having a diameter of from 2.0 to 3.5 mm which is used in the cerical spine for anterior decompression and fusion, and for fixation of a bone graft, (14) a screw-shaped molded article having a diameter of from 3.5 to 4.5 mm which is used in a shaft of a long bone for fixation of a bone graft, (15) a curved or straight pin-shaped molded article having a diameter of from 2.0 to 4.0 mm which is used for osteosynthesis of a fracture of a clavicula or a rib, and (16) a combination of a plate-shaped molded article pierced by holes having a thickness of from 1.0 to 2.0 mm and a screw-shaped molded article having a diameter of from 2.0 to 2.5 mm for fixing said plate-shaped molded article, which is used in oral surgery for osteosynthesis of fractures in a skull, a cheek bone, a nasal bone, a maxilla and a mandibular.

## BRIEF DESCRIPTION OF THE DRAWINGS

Figures 1(a) and 1(b) each show a front view and a plain view, respectively, of a pin according to one embodiment of the present invention.

Figures 2(a) and 2(b) each show a front view and a plain view, respectively, of a plate according to another embodiment of the present invention.

Figure 3 illustrates a perspective view showing a usage of the plate of Fig. 2.

Figure 4(a) is a front view of a screw according to a still another embodiment of the present invention which is suitable for cortical bones.

Figure 4(b) shows an enlarged view of the head of the screw of Fig. 4(a) with an enlarged partial view of the stem thereof (inclusive of the core and the threads).

Figure 5 is a schematic cross section showing a usage of the screw of Fig. 4.

Figure 6(a) is a front view of a screw for compressive retention (malleolar screw).

Figure 6(b) shows an enlarged view of the head of the screw of Fig. 6(a) with an enlarged partial view of the stem thereof (inclusive of the core and the threads).

Figure 7 is a schematic cross section showing a usage of the screw of Figure 6.

Figure 8 is a graph of bending strength and bending modulus vs. stretch ratio of a polylactic acid rod according to the present invention.

Figure 9 is a graph showing changes in bending strength of polylactic acid rode of various sizes with time.

Figure 10 is a graph showing changes in bending strength of polylactic acid rods implanted in various sites with time.

Figure 11 is a graph showing changes in weight of an intra-osseously implanted polylactic acid rod with time.

## DETAILED DESCRIPTION OF THE INVENTION

The molded article according to the present invention can be obtained as follows. Polylactic acid having a viscosity average molecular weight of more than 300,000 is melt-molded by, for example, extrusion molding or press molding at a molding temperature of from the melting point thereof to 220°C to obtain a molded article having a viscosity average molecular weight of from 200,000 to 500,000 and then stretching the molded article at a temperature of from 60° to 160°C or, in order to inhibit molecular weight reduction due to oxidative decomposition, in a nitrogen atmosphere or in an oil. The resulting polylactic acid molded and stretched article is strong and tough as having a bending strength in compression of from $16.0 \times 10^2$ to $25.0 \times 10^2$ kg/cm$^2$ and a bending modulus in compression of from $5.5 \times 10^2$ to $24.0 \times 10^2$ kg/mm$^2$. The polylactic acid molded article having the above-specified physical properties is then formed to have desired shape (e.g., a rod, a plate, a screw, and a pin) and size suitable for curing a fracture at a specific site taking biohydrolyzability and strength as required at that site into consideration.

Crystallinity of polylactic acid can be increased by heat treatment. Crystalline polylactic acid has higher bending strength and higher elastic modulus than non-crystalline polylactic acid. Besides, a crystalline phase is less infiltrated with body fluids (water contents) so that it has a low apparent rate of hydrolysis. The higher the molecular weight of the crystalline polymer, the higher the mechanical properties. However, as the degree of crystallinity is increased by heat treatment, the initial strength is improved but, in turn, polylactic acid is deteriorated due to thermal lability, resulting in a reduction of molecular weight. It follows that the rate of hydrolysis is increased, and deterioration in strength drastically takes place. In other words, a molecular weight and a degree of crystallinity of the polylactic acid should be properly controlled so as to provide a biodegradable and resorbable surgical material having sufficient mechanical properties and hydrolysis resistance for use as a surgical material for osteosynthesis and bone fixation.

The polylactic acid which can be used in the present invention will be explained below in more detail.

Polylactic acid can be synthesized by preparing a lactide of optically active L- or D-lactic acid (i.e., a cyclic dimer of lactic acid) in a usual manner (reference can be made in U.S. Patent 2,668,182 to C.E. Love) and then subjecting the resulting lactide to ring-open polymerization. Since the resulting polylactic acid is poor in thermal stability, it is required to have a viscosity average molecular weight of at least 300,000 taking the molecular weight reduction during subsequent melt-molding into account. The higher the molecular weight, the more suitable for obtaining a high strength surgical material. However, too a high molecular weight necessitates a high temperature and high pressure condition for melt-molding, which causes a great reduction in molecular weight, only to provide a molded article having a molecular weight below 200,000, resulting in a failure of obtaining a desired high strength material. Accordingly, it is suitable to use polylactic acid having a viscosity average molecular weight of from about 300,000 to 600,000, preferably from 350,000 to 550,000, and more preferably from 400,000 to 500,000, as a molding material.

The surgical material according to the present invention can be produced by subjecting the above-described polylactic acid to melt-molding, such as extrusion molding and press molding, into any shape suited to the end use and then uniaxially stretching the molded article in a longitudinal direction. If necessary, the stretched molded article is cut and shaped to obtain a final product having an irregular contour, such as a screw.

Melt-molding having good productivity can be carried out by means of an ordinary extrusion molding machine under the following temperature and pressure conditions.

Melt-molding should be conducted at a temperature ranging from the melting point of polylactic acid to 220°C. At temperatures lower than the melting point, melt extrusion is difficult. At temperatures higher than 220°C, polylactic acid undergoes considerable reduction in molecular weight due to thermal lability only to provide a molded article having a viscosity average molecular weight of less than 200,000.

The molecular weight of the melt-molded article should have a molecular weight of from 200,000 to 500,000, and preferably of from 250,000 to 400,000. If it is less than 200,000, desired improvements in mechanical properties cannot be obtained even after subsequent stretching. If it is more than 500,000, it is difficult to obtain a homogenous molded article. Even though such an article is obtained, decomposition and resorption of the article become slow in a living body more than necessity, though the initial strength is high. If it remains more than necessity, it retards restoration of the bone, and it delays to recover the strength of the bone accordingly. In order to minimize a reduction in molecular weight during melt-molding, it is important to conduct melt-molding at a temperature slightly higher than the melting point of the molding polymer. Therefore, where the polylactic acid to be molded has a molecular weight of from about 400,000 to about 500,000, melt extrusion is preferably carried out at a temperature not higher than 200°C, The higher the molecular weight of the resulting molded article, the better from the viewpoint of mechanical strength.

Likewise, it is preferable to carry out the melt-molding at a possible minimum pressure permitting extrusion which depends on the viscosity (molecular weight) of the polylactic acid in order to minimize a reduction in molecular weight during molding. Accordingly, a suitable extrusion pressure is 260 kg/cm$^2$ or less in the case of the polymer having a molecular weight of up to 600,000 and from about 170 to about 210 kg/cm$^2$ in the case of the polymer having a molecular weight of from 400,000 to 500,000.

Prior to melt extrusion, it is desirable to sufficiently remove a water content from the polymer pellets to be molded by heating under reduced pressure.

Having a viscosity average molecular weight of 200,000 or higher, the melt-extruded article exhibits considerable strength as such but is still insufficient for the purpose. The molded article is then subjected to unidirectional stretching in a longitudinal direction (extrusion direction) in liquid paraffin or a silicon oil or in a heated nitrogen stream. By the stretching, the polymer molecules are orientated to have improved strength.

The degree of crystallinity of the molded article may be increased by heating during stretching. It should be noted, however, that such a heat treatment brings about an improvement in initial strength but causes a reduction in molecular weight. As a result, the resulting product would have an accelerated rate of hydrolysis, and the duration of the strength would be shorter than that of non-crystalline materials. Accordingly, the temperature during stretching is preferably controlled between 60°C and 160°C. Temperatures less than 60°C are unfavorable because of closeness to a glass transition temperature. Stretching at a temperature higher than 160°C, particularly higher than 180°C not only causes a reduction in molecular weight but preferentially induces slide deformation among molecules, failing to achieve molecular orientation nor yet to improve strength. The heating time is preferably within 10 minutes. By stretching, it becomes possible to obtain a molded article having no distortion.

A stretch ratio is preferably between 2 and 6. If it is less than 2, molecular orientation achieved is insufficient for obtaining a satisfactory improvement in strength. On the other hand, a stretch ratio higher than 6 causes fibrillation, resulting in a reduction in hydrolysis resistance.

The thus produced article possesses biodegradability and resorbability and, therefore, there is no fear for the article, when implanted in a living body, to give any adverse influences upon the living body as is observed in using conventional metallic surgical materials. In addition, since the possible reduction in molecular weight during melt molding is minimized, the resulting article maintains its viscosity average molecular weight of from 200,000 to 500,000. Further, the article has had its molecules orientated and its crystallinity increased by stretching. That is, the surgical material according to the present invention exhibits high strength as having a bending strength in compression of from 16.0 x 10$^2$ to 25.0 x 10$^2$ kg/cm$^2$, preferably 17.0 x 10$^2$ to 23.0 x 10$^2$ kg/cm$^2$, a bending modulus in compression of from 5.5 x 10$^2$ to 24.0 x 10$^2$ kg/mm$^2$, preferably 6.0 x 10$^2$ to 20 x 10$^2$ kg/mm$^2$, and a degree of crystallinity of from 10 to 60%, preferably 20 to 40%.

Based on the received clinical knowledge in orthopedics, times required for a damaged or fractured bone in various sites to restore its strength and functions to such an extent that a material implanted for osteosynthesis or fixation may be removed are shown in Table 1 below.

TABLE 1

| Site | Required Duration of Strength Retention (week) |
|---|---|
| Shoulder joint | 6 - 8 |
| Elbow joint | 6 |
| Wrist joint and fingers | 6 - 12 |
| Pelvis to hip-joint | 6 - 8 |
| Knee joint | 6 - 12 |
| Ankle joint | 6 - 16 |
| Toes | 6 - 8 |
| Cerical spine | 4 |
| Shaft of long bones | 8 |
| Skull | 6 |
| Sternum and ribs | 6 - 8 |

The above table tells how long the polylactic acid molded article of the present invention, when implanted in a human body at a specific site, should retain its strength. The term "strength" as used herein does not mean 100% strength. The biodegradable polylactic acid undergoes hydrolysis in vivo at its ester linkage to have its molecular weight reduced. In actual use, hydrolysis of the polylactic acid molded article starts from the surface of the article in contact with body fluids and then gradually proceeds into the inside of the article to cause cracking, leading to deterioration in strength of the whole article. Therefore, there is a period in which the article undergoes substantially no apparent change in strength, followed by a short period in which the strength is gradually reduced, finally followed by an abrupt reduction in strength. A damaged or fractured bone is on towards recovery during such a period of strength reduction. Namely, since the degradation of the polylactic acid molded article is compensated by the recovery of the bone, the molded article is not necessarily required to 100% retain its strength. It is rather desirable that most of the strength of the molded article be lost immediately after the period of recovery so as not to give any burden on the bone. From this viewpoint, an implanted molded article should be deteriorated in strength within 4 to 16 weeks from implantation. In other words, with differences between individuals being taken into consideration, it is necessary that the molecular weight and stretch ratio of the polylactic acid and the size and thickness of the polylactic acid molded article be selected so that the molded article should retain at least about 30%, and preferably at least 50%, of the initial strength in the final stage of recovery.

In general, when a polymer is used as a structural material for its own strength, deterioration in strength offers no serious problem because the rate of deterioration is so low that there is little need to consider such deterioration.

However, in the cases where biodegradability and resorbability as possessed by the polylactic acid of the present invention is utilized as one of functions, the rate of degradation and the time when the strength as a structural material is lost are of extreme importance in practical use. In particular, when a structural material is used in a living body, if it has a high rate of degradation and undergoes considerably advanced deterioration in strength in the early stage of healing where regeneration of bone at the site of a fracture is still insufficient, such a material is regarded to be of no practical use.

Hydrolysis of a polyester type biodegradable polymer, such as polylactic acid, is based on decomposition of the ester linkage of its primary structure. While the rate of hydrolysis of such a substance is on the molecular level inherent to the substance, actual degradation which occurs when the substance is embedded in a living body starts from the area in contact with weakly alkaline (pH=7.4) body fluids. That is, the molded article of the present invention having a shape of a screw, a rod, a plate, a pin, etc. starts to undergo hydrolysis from its surface on practical use. Thus, the specific surface area of a molded article is a factor determining deterioration in strength. A molded article having a large surface area for its size (i.e., having a shape in high relief) shows a relatively high rate of deterioration as a whole, while a molded article having a small surface area for its size (i.e., having a shape in low relief) shows a relatively low rate of deterioration.

In particular, in the polylactic acid molded articles of the present invention which have been subjected to stretching to increase strength to the equal level as that of bones, since only slight deterioration easily leads to a drastic reduction in strength as a whole to a fairly lower level than the practically useful level, the specific surface area, i.e., shape and size, of the molded article should be carefully decided. Weight being attached only to strength, a molded article having a large size and a simple shape with an even surface may be used. However, there is a limit in size of a molded article applicable to a specific site of curing. Also, if a molded article has too a large size, there is produced a large amount of degradation products, which entertains a fear of undesired reactions with a living body.

Deterioration in strength due to hydrolysis is initiated by the formation of cracks in the surface of a molded article. Since surface cracks play a roll as notches, observed strength values are reduced. Experience tells that the surface crack is a factor preceding a molecular weight reduction in actual manifestation of strength reduction. While the surface polymer in contact with body fluids suffers from reduction in molecular weight due to hydrolysis, it is not until hydrolysis proceeds deep into the inside of the molded article that cracks appreciable to the eye appear. Namely, deterioration taking place only on the surface layer of the molded article does not mean so much deterioration as a whole. It is thus the most desirable that the time required for hydrolysis to sufficiently proceed to the inside of a molded article to cause cracking, i.e., the time required for actual manifestation of reduction in strength should be substantially the same as the time required for a bone to be regenerated to exhibit strength to some extent. In fact, it is a thickness of a molded article that decides the former. From the fact that the thickness direction of a molded article is the shortest course for body liquids surrounding a molded article to pierce the article irrespective of the shape of the molded article, it can be understood that the thickness of a molded article is the greatest factor determining the time of manifestation of strength reduction even though the shape of an article is a rod, plate, screw, etc. Therefore, even with a unit strength being equal, molded articles having a large specific surface area, e.g., powders, granules, and pins or rods of small diameter, show a higher rate of strength reduction than those having a large thickness, e.g., rods or screws of large diameter and thick plates.

Based on these facts, the inventors experimentally embedded various molded articles of different shape and size in the body of experimental animals and determined a thickness of a molded article which, when implanted in a living body, shows deterioration in strength due to degradation in an adequate stage of healing, thus reaching the present invention. No cases have as yet been reported in which the rate of biodegradation of a polylactic acid molded article is considered in connection with a thickness thereof.

Surgical experience tells that the time required for regeneration of a bone of a human body, i.e., the period in which an implanted polymer should retain its strength ranges from about 4 to about 16 weeks, though varying depending on the site of the bone. In order that a molded article to be used as a surgical material for bone fixation or osteosynthesis may retain its strength on the initial level or at least on a level reduced somewhat but not to an extent interfering with curing during the above-described period, it is required that the thickest portion of the molded article should have a thickness of from about 1.0 to about 4.5 mm.

However, the following points should be taken into consideration in deciding a suitable thickness of molded articles. In the case of a screw-shaped molded article composed of a thick portion and a thin portion, the threads thereof have a small thickness while the core thereof has a large diameter, i.e., a large thickness. When the screw is inserted into a bone, the bending strength of the screw depends on the thickness of the core, with the threads predominantly serving for fixation of fragments of a fractured bone.

When a screw is used for osteosynthesis at a fractured site, a hole in which the screw is turned to enter is previously tapped. Accordingly, the screw is set in such a manner that the tapped hole is filled with the screw. After the implantation, the screw begins to undergo deterioration due to hydrolysis from the surface thereof in contact with body fluids. The deterioration gradually proceeds into the inside of the screw. The threads and the thin part of the tip of the screw undergo deterioration throughout and suffer from surface cracking sooner than other parts. At this point, the strength of these thin parts of the screw is considerably reduced whereas the core of the screw is not so deteriorated as causing interference with osteosynthesis. That is, in the case of screws, the diameter of the core part decides the duration of strength. The term "diameter of a screw" as used herein means a diameter of the core of a screw. In the case of simple-shaped molded articles such as a rod, a pin, and a plate, the thickness thereof decides the duration of strength.

The present invention is now illustrated in greater detail with reference to Examples, but it should be understood that the present invention is not deemed to be limited thereto. In Examples, in vivo tests were carried out using white rabbits to grasp degrees of deterioration of the molded articles according to the present invention in actual use in a living body thereby to corroborate specific sites of bones to which the molded articles can be clinically applied for bone fixation or osteosynthesis.

Physical properties of molded articles obtained were measured as follows.

1) Bending Strength in Compression:

Measured in accordance with JIS K-7203.

2) Bending modulus in Compression:

Measured in accordance with JIS K-7203.

3) Degree of Crystallinity:

A density of a specimen was measured at 30°C by using a density gradient tube containing an n-hexane/carbon tetrachloride system. Prior to the measurement, the specimen was put in n-hexane for 30 minutes for defoaming.

A degree of crystallinity of the specimen was calculated from the measured density according to formula:

$$1/\rho = X/\rho c - (1 - X)/\rho a$$

wherein X is a degree of crystallinity; $\rho$ is a measured density of a specimen; $\rho c$ is a density of a crystalline phase ($=1.290$ g/cm$^3$); and $\rho a$ is a density of a non-crystalline phase ($=1.248$ g/cm$^2$).

EXAMPLE 1

Preparation of Polymer:

Pellets of polylactic acid having an initial viscosity average molecular weight of 420,000 were dried at 80 to 120°C for one day under reduced pressure. The dried pellets were charged in an extrusion molding machine, allowed to stand under reduced pressure for about 40 minutes, and then extrusion molded at a cylinder temperature of 198°C, an adapter temperature of 200°C, and a die temperature of 200°C to obtain a round bar or a square bar. The resulting molded article had a viscosity average molecular weight of 220,000 as calculated by using relationship: $[\eta] = 5.45 \times 10^{-4}M^{0.73}$ (at 25°C in chloroform).

The resulting molded article was uniaxially stretched in the longitudinal direction at a stretch ratio of 2 in liquid paraffin at 105°C and then cut to prepare specimens of 5.0 mm in diameter and 80 mm in length. The specimen had a bending strength in compression of 1720 kg/cm$^2$, a bending modulus in compression of 610 kg/mm$^2$, and a degree of crystallinity of 28%.

When the specimen was immersed in physiological saline at 37°C for 3 months, the physical properties were 1700 kg/cm$^2$ in bending strength in compression and 600 kg/mm$^2$ in bending modulus in compression, indicating that the specimen underwent substantially no deterioration.

In Vivo Test:

Polylactic acid having an initial viscosity average molecular weight of 420,000 was extrusion molded and then stretched under the same conditions as described above to prepare a round rod having a length of 80 mm and a diameter of 4.5, 4.0, 3.0, 2.0 or 1.0 mm.

Each of the resulting specimens was embedded in the backbone of a white rabbit. The specimen was removed at a prescribed time interval, and the bending strength was measured with time. The results obtained are shown in Table 2 below. Three animals were used for each specimen for each time of measurement, and the three measured values were averaged.

TABLE 2

| Time of Measurement (week) | Bending strength in Compression (kg/cm$^2$) | | | | |
|---|---|---|---|---|---|
| | $\varnothing$4.5 mm | $\varnothing$4.0 mm | $\varnothing$3.0 mm | $\varnothing$2.0 mm | $\varnothing$1.0 mm |
| 0 | 2230 | 2170 | 2200 | 2180 | 2200 |
| 2 | 2270 | 2150 | 2100 | 2090 | 1990 |
| 4 | 2230 | 2100 | 2060 | 1960 | 1760 |
| 6 | 2210 | 2020 | 1930 | 1610 | 700 |
| 8 | 2200 | 1900 | 1580 | 370 | 130 |
| 10 | 2160 | 1750 | 690 | 140 | 100 |
| 12 | 2000 | 1600 | 320 | 100 | 60 |
| 14 | 1950 | 900 | 160 | 90 | 50 |
| 16 | 1750 | 850 | 130 | 60 | 30 |

The rate of strength reduction of the specimens implanted in the backbone of white rabbits is subject to variation depending on differences among individuals, the state of fixing the specimen to the bone, the adhesion of the specimen to the bone, and the stress imposed to the specimen during the test, and the like. The rate of regeneration of the damaged bone also varies among individuals due to a difference in recuperative power. With all these variations being taken into consideration, the surgical material, while being implanted, must retain at least 30%, and preferably 50% or more, of the initial strength during the period of recovery specified according to the site as shown in Table 1.

Validity of Thickness of Molded Article:

In order to examine validity of thickness of molded articles, the time period in which about 70% of the initial strength was maintained (hereinafter referred to as 70% retention time) was determined for each specimen from the curve of the strength vs. time plots. From the thus obtained 70% retention time in view of the data shown in Table 1, the following conclusions were drawn.

A curved or straight pin having a thickness or a diameter of from 2.0 to 4.0 mm had a 70% retention time of from 6 to 12 weeks and, accordingly, proved suitable for use in osteosynthesis of fractured breast bones or rib bones.

A screw having a diameter of from 2.0 to 4.0 mm had a 70% retention time of from 6 to 12 weeks and, accordingly, proved suitable for use in a shoulder joint for fixation of the greater tuberosity, osteoclastic fragments of the collum chirurgicum, and fragments of glenoid.

A pin having a diameter of from 1.0 to 2.5 mm had a 70% retention time of from 5 to 8 weeks and, accordingly, proved suitable for use in an elbow joint for osteochondral fracture or fixation in osteotomy or bone grafting for the treatment of baseball elbow.

A screw having a diameter of from 2.0 to 3.0 mm had a 70% retention time of from 6 to 9 weeks and, accordingly, proved suitable for use in an elbow joint for osteosynthesis of a fractured medial epicondyle of humerus or lateral of epicondyle of humerus.

A pin having a diameter of from 1.0 to 2.0 mm had a 70% retention time of from 5 to 8 weeks and, accordingly, proved suitable for use in carpal bones and phalanx of finger for osteosynthesis of fractured phalanx of finger at various sites, intraarticular fractured bones, a fractured distal radius, etc.

A microscrew having a diameter of from 1.5 to 2.5 mm had a 70% retention time of from 6 to 8 weeks and, accordingly, proved suitable for use in the wrist joint and phalax of finger for osteosynthesis of a fractured distal radius, fractured phalax of finger, etc.

A pin or screw having a diameter of from 2.5 to 3.5 mm had a 70% retention time of from 8 to 11 weeks and, accordingly, proved suitable for use in the pelvis and the hip-joint for osteosynthesis of a fractured acetabulum or an avulsion fractured ilium, fixation of a graft in arthroplasty, and for osteosynthesis of a fractured femoral head.

A pin or screw having a diameter of from 3.5 to 4.5 mm had a 50% retention time of from 11 to 16 weeks and, accordingly, proved suitable for use in a knee joint for osteosynthesis of a fractured femoral condyle.

A pin or screw having a diameter of from 2.5 to 3.5 mm had a 70% retention time of from 8 to 11 weeks and, accordingly, proved suitable for use in a knee joint for osteosynthesis of intraarticular fractured bones, an osteochondritis dissecans, or fractured proximal tibia or osteosynthesis in osteotomy.

A screw having a diameter of from 2.5 to 3.5 mm had a 70% retention time of from 8 to 11 weeks and, accordingly, proved suitable for use in an ankle joint for osteosynthesis of a fractured medial, lateral or posterior malleolus.

A screw having a diameter of from 3.5 to 4.5 mm had a 70% retention time of from 11 to 16 weeks and, accordingly, proved suitable for use in an ankle joint for osteosynthesis of a fractured talus.

A pin having a diameter of from 2.0 to 4.0 mm had a 70% retention time of from 6 to 12 weeks and, accordingly, proved suitable for use in an ankle joint for osteosynthesis of an osteochondritis dissecans and an osteochondral fracture.

A pin having a diameter of from 1.5 to 3.0 mm had a 70% retention time of from 6 to 9 week and, accordingly, proved suitable for use in toes for osteosynthesis in operation of hallux valgus and osteosynthesis of fractured phalanx of toe.

A screw having a diameter of from 2.0 to 3.5 mm had a 70% retention time of from 6 to 11 weeks and, accordingly, proved suitable for use in the cerical spine for anterior decompression and fusion, and for fixation of a bone graft.

A screw having a diameter of from 3.5 to 4.5 mm had a 50% retention time of from 11 to 16 weeks and, accordingly, proved suitable for use in a shaft of a long bone for fixation of a bone graft.

A combination of a plate having a thickness of from 1.0 to 2.0 mm and a screw having a diameter of from 2.0 to 2.5 mm for fixing the plate had a 70% retention time of from 5 to 8 weeks and, accordingly, proved suitable for use as a material for oral surgery for osteosynthesis of fractured skull, cheek bone, nasal bone, maxilla, and mandibula.

Since the tests were carried out in vivo, the strength deterioration rate is varied a little dependent on an individual and a region, as a matter of course.

The present invention is further described in detail by referring to the accompanying drawings.

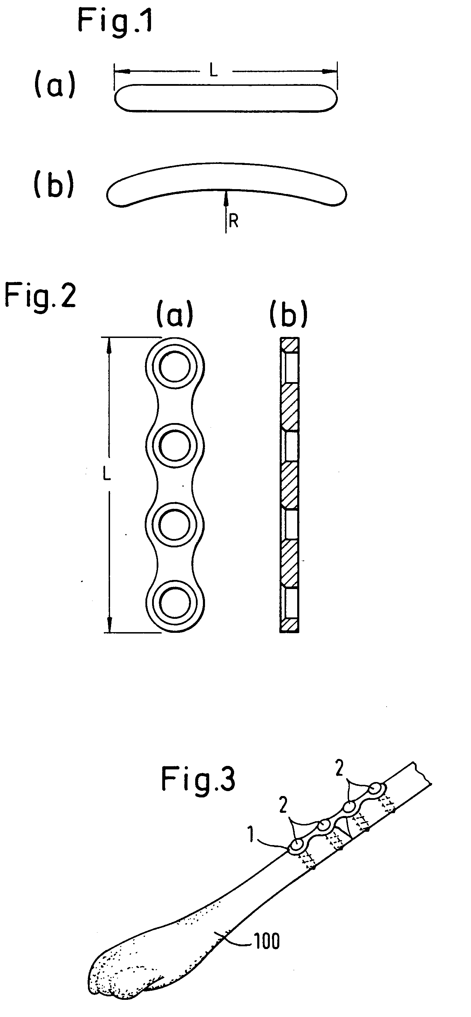

Fig. 1 illustrates a front view (a) and a plain view (b) of a pin according to the present invention. The pin has a length (L) of 37.5 mm and has a 3 to 4 mm-square section with a curved surface having a curvature radius (R) of 50. The pin of Fig. 1 is used for fixation of rib bones, and the requisite duration of strength for that use is from 6 to 8 weeks.

Fig. 2 illustrates a front view (a) and a side view (b) of a plate according to the present invention. The plate is 22.6 mm long (L) and has four small holes. The plate of this type is used for curative application of pressure to fractured ends of a bone to accelerate healing.

Fig. 3 schematically illustrates a usage of the plate of Fig. 2. In Fig. 3, plate 1 is fixed to fractured bone 100 by means of screws 2 screwed into the small holes of plate 1. The plate-shaped molded articles according to the present invention include other various embodiments, and the most suitable type is chosen according to the condition or the site of a fracture.

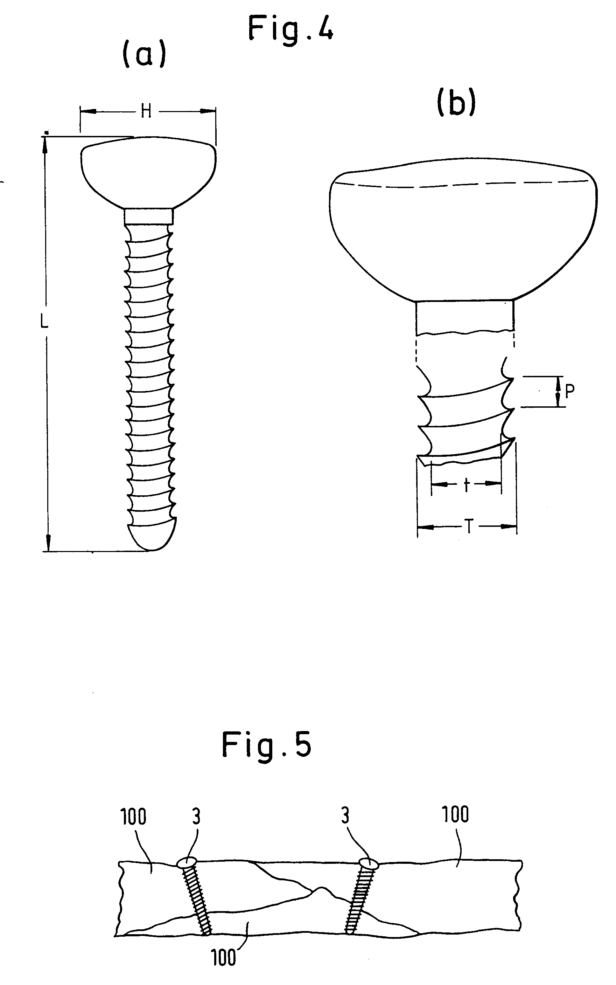

Fig. 4(a) illustrates a screw for cortical bones. The screw for cortical bones has a large channel width and a length (L) of 14 to 70 mm with the head of 7.5 mm in diameter (H). It is often used for compressive reposition and fixation in oblique fractures or in combination with a plate.

Fig. 4(b) illustrates an enlarged view of the head and a partial enlarged view of the threads of the screw of Fig. 4(a). The pitch (P) is 1.75 mm, and the minimum diameter (t) and the maximum diameter (T) of the stem portion are 3.0 mm and 4.5 mm, respectively. The screw of this type includes another embodiment having an H of 5.5 mm, an L of from 10 to 40 mm, a P of 1.75 mm, a t of 1.9 mm, and a T of 3.5 mm and still another embodiment having an H of 3.0 mm, an L of from 5 to 15 mm, a P of 1.8 mm, a t of 1.4 mm, and a T of 2.0 mm, etc.

Fig. 5 schematically illustrates a usage of the screw for cortical bones. The numeral 100 indicates a bone, and the numeral 3 indicates the screw.

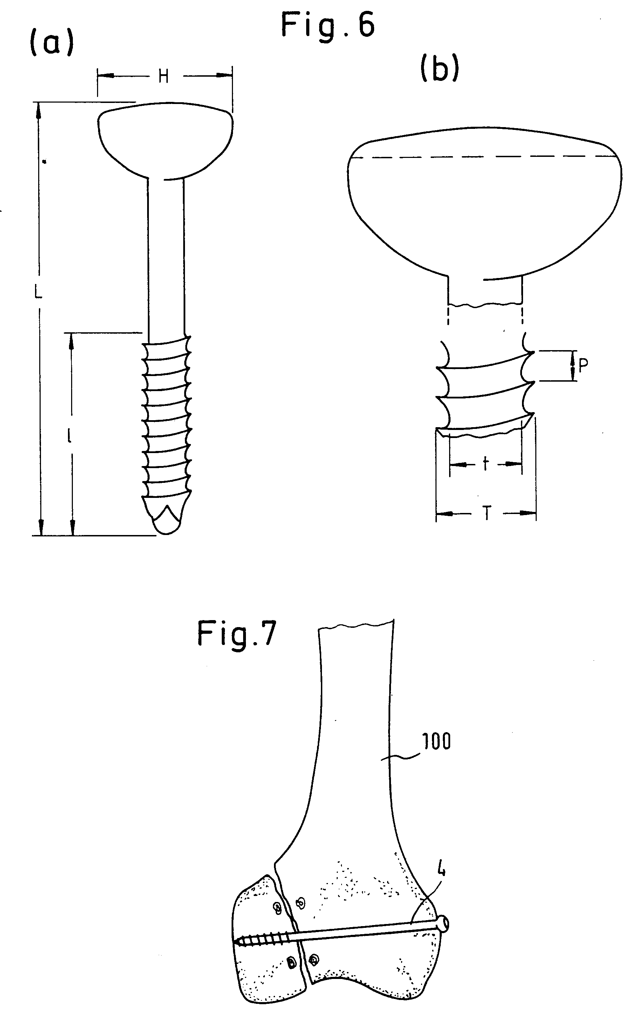

Fig. 6(a) shows a screw for compressive reposition and fixation called a malleolar screw. The broad and sharp threads hold cancellus bones similarly to the screw for cortical bones. It is so designed that the tissue may not be caught by the head portion of the screw when the screw is obliquely inserted. Fig. 6(b) shows an enlarged view of the head and a partial enlarged view of the threads of the malleolar screw. The screw had an H of 7.5 mm and an L of from 25 to 70 mm, and the screw part thereof has a length ($\ell$) of from 12 to 32 mm, a t of 3.0 mm, and a T of 4.5 mm.

Fig. 7 shows a usage of the malleolar screw, in which the numeral 100 indicates a bone, and the numeral 4 indicates a malleolar screw.

As can be understood from the foregoing, the biodegradable and resorbable surgical molded articles according to the present invention retain their strength until a damaged or fractured bone is gradually regenerated to restore its strength to an extent permitting of daily living and is then degraded and resorbed into a living body by the time osteogenesis substantially completes. Further, in the present invention, the polylactic acid molded article is so specified as to have the optimum size and shape for a specific site of bones to which it is applied thereby to have a balance between strength required for fixation or setting of bones and its biodegradability and resorbability. The above-described effects of the present invention can thus be manifested pronouncedly.

## EXAMPLE 2

Behaviors of rods prepared from the high strength polylactic acid of the present invention were examined chiefly for their strength in vitro and in vivo.

### In vitro Test:

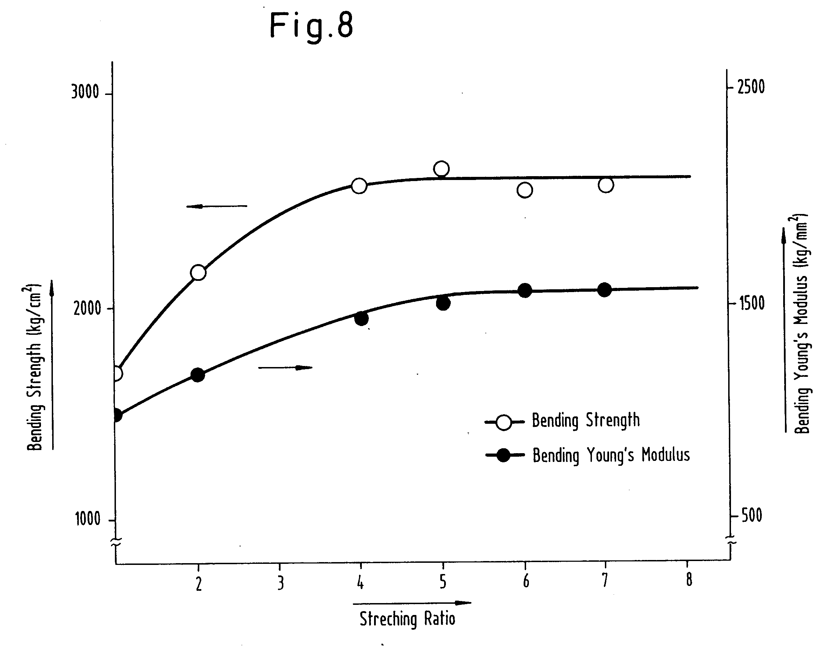

An extruded (after pre-drying at 90°C under vacuum) rod of polylactic acid (viscosity average molecular weight: 210,000) having a diameter of 3.2 mm was uniaxially stretched (2.5 times at 100°C in liquid paraffin) at a varied stretch ratio, and a change in bending strength and bending modulus with a stretch ratio was determined. The results obtained are shown in Fig. 8.

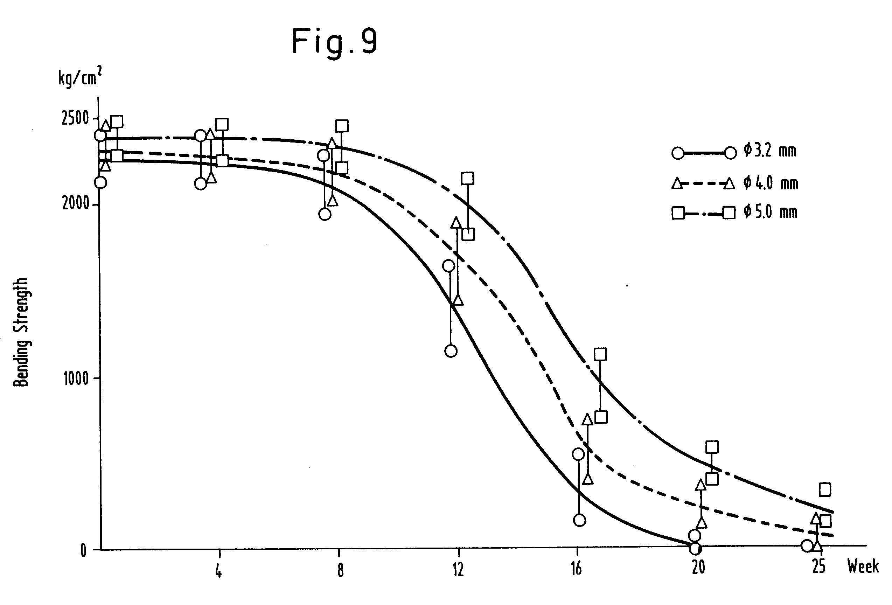

Then, a polylactic acid rod having a diameter of 3.2 mm or 4.5 mm was uniaxially stretched at a stretch ratio of 2.5. The stretched rod was subjected to hydrolysis in a phosphoric acid buffer solution at 37°C, and a change in bending strength with time was determined. The results obtained are shown in Fig. 9.

### 2) In Vivo Test:

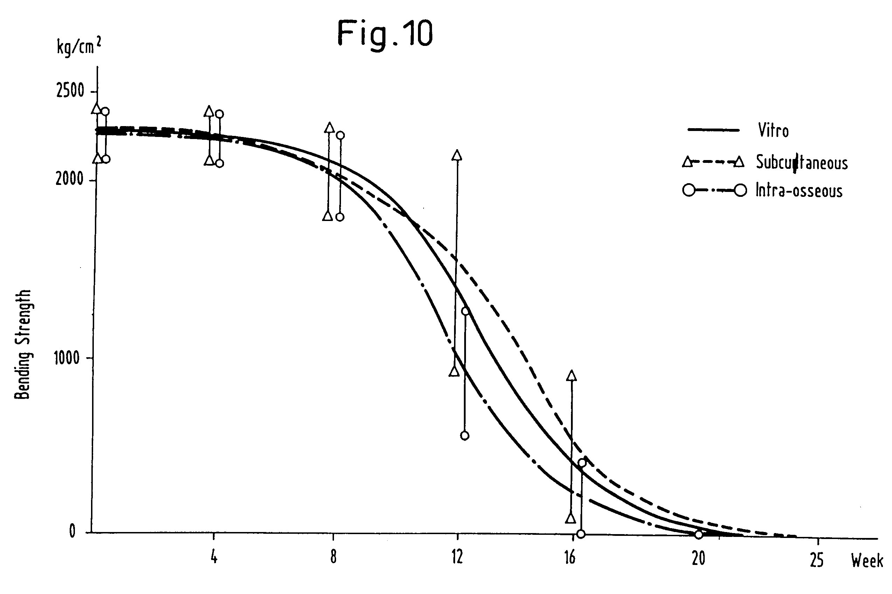

Five polylactic acid rods having a diameter of 3.2 mm and a length of 5 cm were subcutaneously implanted in each of 10 domestic rabbits. Further, a rod of the same size was intra-osseously implanted into each femur from the respective knee joint of each of 20 domestic rabbits like an intramedullary nail. The rabbits were killed after 4, 8, 12, 16, 25 and 52 weeks from the subcutaneous or intra-osseous implantation, and changes in strength and weight of the rod, and tissue with time were examined. The results obtained are shown in Figs 10 to 13.

### Results:

The strength vs. stretch ratio plots of Fig. 8 show that bending strength linearly increased with an increase in stretch ratio up to 3 and came to substantial equilibrium at a stretch ratio of 4 where it reached about 1.8 times (2700 kg/cm$^2$) that of the non-stretched rod. Bending modulus also increased to 1600 kg/mm$^2$ (about twice that of the non-stretched rod).

The graph of Fig. 9 shows that the rod of 3.2 mm in diameter retained its initial strength (2200 kg/cm$^2$) for 8 weeks, underwent a reduction to 60% and 20% of the initial strength due to 12 weeks' and 16 weeks' hydrolysis, respectively, and almost lost its strength in 25 weeks. The larger the size of the rod, the smaller the degree of strength reduction due to hydrolysis.

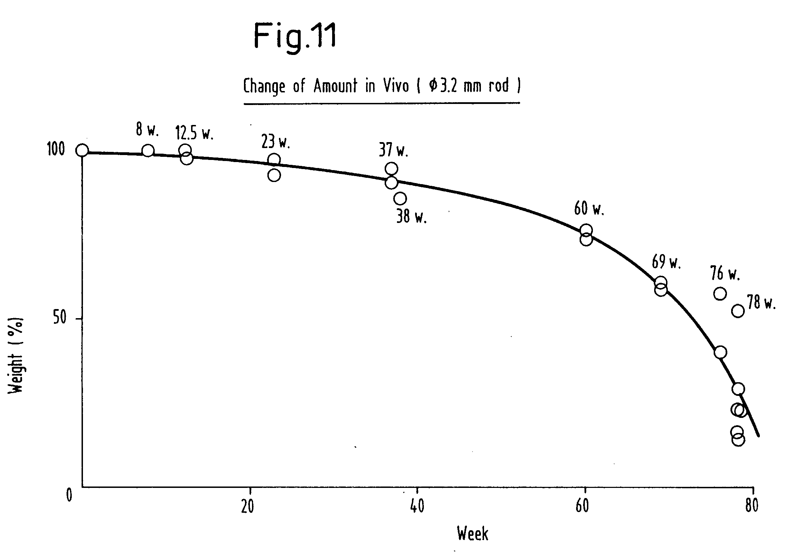

As can be seen from the graph shown in Fig. 10, the change in strength in the subcutaneous site was substantially the same as was observed in the in vitro test, though showing a large scatter among individuals. The strength reduction tended to be slightly suppressed in the intra-osseous test during the period of from 8 to 12 weeks from the embedding as compared with the in vitro test. The intra-osseously embedded rod was reduced in weight by about 5% in 25 weeks and by about 20 to 30% in 52 weeks as shown in Fig. 11.

In the intra-osseous test, active intermediate osteogenesis was observed around the rod from about 8 weeks from the embedding, and a giant cell against a foreign body was observed in just a small part of the tissue after 16 weeks. A fibrous tissue was observed to enter into the crack formed in the rod in a part of the tissue after 25 weeks. After 52 weeks from the embedding, medulla was formed in the bone to form a double ring but no macrophage was observed.

At least 50% of the initial strength was maintained within about 4 to 16 weeks. Thereafter, gradual decomposition was proceeded to decrease the strength and the molecular weight. In about 20 to 30 weeks, the strength was disappeared. Gradual decomposition was further proceeded and the molecular weight was further lowered. From about 40 weeks, resorption was accelerated. On about 1.5 years (78 weeks), at least the half of the initial amount was resorbed into a living body.

In the above-described experiments, a strength higher than that of human cortical bones was obtained by stretching at a stretch ratio of 2 or more, and such a strength could be maintained for a period of from 8 to 12 weeks in a bone. Accordingly, the rod under test proved to suffice for use in osteosynthesis for fractures in a human body which is usually healed in 2 to 3 months. The fact that a rod of greater size is slower in deterioration appears advantageous in clinical use.

While the invention has been described in detail and with reference to specific examples thereof, it will be apparent to one skilled in the art that various changes and modifications can be made therein without departing from the scope of the claims.

## Claims

1.  A biodegradable and resorbable molded article for surgery which comprises a polylactic acid molded and stretched article having a viscosity average molecular weight of from 200,000 to 500,000, a bending strength in compression of from $16.0 \times 10^2$ to $25.0 \times 10^2$ kg/cm$^2$, a bending modulus in compression of from $5.5 \times 10^2$ to $24.0 \times 10^2$ kg/mm$^2$, and a degree of crystallinity of from 10 to 60% as calculated from density, the thickest portion of said molded and stretched article being controlled between 1.0 mm and 4.5 mm so that said article is deteriorated in strength due to degradation in a living body within about 4 to 16 weeks and resorbed into a living body at least 50% during from about 1.5 to 2.0 years.

2.  Molded article as claimed in Claim 1, characterized in that said article is a screw-shaped molded article.

3.  Molded article according to claim 2, characterized in that said article has a diameter of from 2.5 to 3.5 mm.

4.  Use of a molded article according to claim 3 for preparing a shoulder joint for fixation of the greater tubeorsity, osteoclastic fragments of the collum chirurgium, and fragments of the glenoid;
    or for preparing a pelvis and/or hip-joint for osteosynthesis of a fracture of the acetabulum and an avulsion fracture of the ilium for fixation of a graft in hip-joint arthroplasty, and for osteosynthesis of a fracture of the femoral head;
    or for preparing a means for osteosynthesis of an intraarticular fracture, an osteochondritis dissecans, a fracture of the proximal tibia or for osteosynthesis in osteotomy;
    or for preparing a means for osteosynthesis of a fracture in the medial, lateral or posterior malleolus of an ankle joint.

5.  Molded article according to claim 2, characterized in that said article has a diameter of from 2.0 to 3.0 mm.

6.  Use of a molded article according to claim 5 for preparing an elbow joint for osteosynthesis of a fracture of the medial epicondyle of humerus or lateral epicondyle of humerus.

7.  Molded article according to claim 2, characterized in that said article has a diameter of from 3.5 to 4.5 mm.

8.  Use of a molded article according to claim 7 for preparing a knee joint for osteosynthesis of a fracture of the femoral condyle;
    or for preparing a means for osteosynthesis of a fracture of the talus in an ankle joint;
    or for preparing a means for use in a shaft of a long bone for fixation of a bone graft.

9.  Molded article according to claim 2, characterized in that said article has a diameter of from 2.0 to 3.5 mm.

10. Use of a molded article according to claim 9 for preparing a means for use in a cervical spine for anterior decompression and fusion, and for fixation of a bone graft.

11. Molded article as claimed in claim 1, characterized in that said article is a pin-shaped molded article.

12. Molded article as claimed in claim 11, characterized in that said article has a diameter of from 1.0 to 2.0 mm.

13. Use of a molded article according to claim 12 for preparing a means for use in carpal bones and metacarpals for osteosynthesis of a fracture of phalanx of finger in various sites, and intraarticular fracture, a fracture of distal radius.

14. Molded article according to claim 11, characterized in that said article has a diameter of from 1.0 to 2.5 mm.

15. Use of a molded article according to claim 14 for praparing an elbow joint for osteochondral fracture and fixation of a bone graft for curing baseball elbow.

16. Molded article according to claim 11, characterized in that said article has a diameter of from 2.5 to 3.5 mm.

17. Use of a molded article according to claim 16 for preparing a pelvis and/or hip-joint for osteosynthesis of a fracture of the acetabulum and an avulsion fracture of the ilium, for fixation of a graft in hip-joint arthroplasty, and for osteosynthesis of a fracture of the femoral head;
or for preparing a knee joint for osteosynthesis of an intraarticular fracture, an osteochondritis dissecans, a fracture of the proximal tibia or for osteosynthesis in osteotomy.

18. Molded article according to claim 11, characterized in that said article has a diameter of from 2.0 to 4.0 mm.

19. Use of a molded article according to claim 18 for preparing a means used in an ankle joint for osteosynthesis of an osteochondritis dissecans and an osteochondral fracture.

20. Molded article according to claim 11, characterized in that said article has a diameter of from 3.5 to 4.5 mm.

21. Use of a molded article according to claim 20 for preparing a knee joint for osteosynthesis of a fracture of the femoral condyle.

22. Molded article according to claim 11, characterized in that said article has a diameter of from 1.5 to 2.5 mm.

23. Use of a molded article according to claim 22 for preparing a means for use in toes in an operation of a hallux valgus or osteosynthesis of a fracture of a phalanx of toe.

24. Molded article according to claim 1, characterized in that said article is a microscrew-shaped molded article.

25. Molded article according to claim 24, characterized in that said article has a diameter of from 1.5 to 2.5 mm.

26. Use of a molded article according to claim 25 for preparing a means used in a wrist joint and a phalax of finger for osteosynthesis of a fracture of a distal radius and a fracture of phalax of finger.

27. Molded article according to claim 1, characterized in that said article is a curved or straight rod-shaped molded article.

28. Molded article according to claim 27, characterized in that said article has a diameter of from 2.0 to 4.0 mm.

29. Use of a molded article according to claim 28 for preparing a means for osteosynthesis of a fracture of a the clavicula or the rib.

30. Molded article according to claim 1, characterized in that said article is a combination of a plate-shaped molded article pierced by holes having a thickness of from 1.0 to 2.0 mm and a screw-shaped molded article having a diameter of from 2.0 to 2.5 mm for fixing said plate-shaped molded article.

31. Use of a molded article according to claim 30 for preparing a means used in oral surgery for osteosynthesis of fractures in the skull, the cheek bone, the nasal bone, the maxilla, and the mandibular.

**Patentansprüche**

1. Biologisch abbaubares und resorbierbares Formteil für die Chirurgie, das ein geformtes und gedehntes Teil aus Polymilchsäure umfaßt, das ein Viskositätsmittel des Molekulargewichts zwischen 200.000 und 500.000; eine Biegefestigkeit bei Kompression zwischen $16,0 \times 10^2$ und $25,0 \times 10^2$ kg/cm$^2$; ein Biegemodul bei Kompression zwischen $5,5 \times 10^2$ und $24,0 \times 10^2$ kg/mm$^2$ und einen Grad der Kristallinität von 10 bis 60 %, aus der Dichte errechnet, aufweist; wobei der dickste Bereich des gedehnten und geformten Teils auf zwischen 1,0 mm und 4,5 mm kontrolliert ist, so daß das Teil sich aufgrund eines Abbaus in einem lebenden Körper innerhalb von etwa 4 bis 16 Wochen in seiner Festigkeit verschlechtert, und in einem lebenden Körper im Verlauf von etwa 1,5 bis 2,0 Jahren zu mindestens 50 % resorbiert ist.

2. Formteil nach Anspruch 1, dadurch **gekennzeichnet,** daß das Teil ein schraubenförmiges Formteil ist.

3. Formteil nach Anspruch 2, dadurch **gekennzeichnet,** daß das Teil einen Durchmesser von 2,5 bis 3,5 mm hat.

4. Verwendung eines Formteils nach Anspruch 3 zur Herstellung eines Schultergelenks zur Fixierung des größeren Tuber, von osteoklastischen Fragmenten des chirurgischen Halses und glenoidalen Fragmenten; oder zur Herstellung eines Beckens und/oder Hüftgelenks zur Osteosynthese einer Fraktur des Azetabulums und einer Abrißfraktur des Iliums, zur Fixierung eines Transplantats in der Hüftgelenk-Arthroplastik und zur Osteosynthese einer Fraktur des Femurkopfes; oder zur Herstellung eines Mittels zur Osteosynthese einer intraartikulären Fraktur, von Osteochondritis dissecans, einer Fraktur der proximalen Tibia oder zur Osteosynthese in der Osteotomie; oder zur Herstellung eines Mittels zur Osteosynthese einer Fraktur des medialen, lateralen oder hinteren Malleolus eines Knöchelgelenks.

5. Formteil nach Anspruch 2, dadurch **gekennzeichnet,** daß das Teil einen Durchmesser von 2,0 bis 3,0 mm hat.

6. Verwendung eines Formteils nach Anspruch 5 zur Herstellung eines Ellbogengelenks zur Osteosynthese einer Fraktur des medialen Oberarm-Epicondylus oder des lateralen Oberarm-Epicondylus.

7. Formteil nach Anspruch 2, dadurch **gekennzeichnet,** daß das Teil einen Durchmesser von 2,5 bis 4,5 mm hat.

8. Verwendung eines Formteils nach Anspruch 7 zur Herstellung eines Kniegelenks zur Osteosynthese einer Fraktur des femoralen Condylus; oder zur Herstellung eines Mittels zur Osteosynthese einer Fraktur des Talus in einem Sprunggelenk; oder zur Herstellung von Mitteln zur Verwendung in einem Schaft eines langen Knochens zur Fixierung eines Knochentransplantats.

9. Formteil nach Anspruch 2, dadurch **gekennzeichnet,** daß das Teil einen Durchmesser von 2,0 bis 3,5 mm hat.

10. Verwendung eines Formteils nach Anspruch 9 zur Herstellung von Mitteln zur Verwendung in einer zervikalen Spina für eine anteriore Dekompression und Fusion und zur Fixierung eines Knochentransplantats.

11. Formteil nach Anspruch 1, dadurch **gekennzeichnet,** daß das Formteil ein stiftförmiges Formteil ist.

12. Formteil nach Anspruch 1, dadurch **gekennzeichnet,** daß das Formteil einen Durchmesser von 1,0 bis 2,0 mm hat.

13. Verwendung eines Formteils nach Anspruch 12 zur Herstellung eines Mittels zur Verwendung in karpalen Knochen und metacarpalen Knochen zur Osteosynthese einer Fraktur der Fingerphalanx an verschiedenen Stellen sowie einer intraartikulären Fraktur, einer Fraktur der distalen Speiche.

14. Formteil nach Anspruch 11, dadurch **gekennzeichnet,** daß das Formteil einen Durchmesser von 1,0 bis 2,5 mm hat.

15. Verwendung eines Formteils nach Anspruch 14 zur Herstellung eines Ellenbogengelenks für eine osteochondrale Fraktur und zur Fixierung eines Knochentransplantats zur Heilung eines Baseball-Ellenbogens.

16. Formteil nach Anspruch 11, dadurch **gekennzeichnet,** daß das Formteil einen Durchmesser von 2,5 bis 3,5 mm hat.

17. Verwendung eines Formteils nach Anspruch 16 zur Herstellung eines Beckens und/oder Hüftgelenks für eine Osteosynthese einer Fraktur des Azetabulums und einer Abrißfraktur des Ilium, zur Fixierung eines Transplantats in der Hüftgelenk-Arthroplastik sowie zur Osteosynthese einer Fraktur des Femurkopfes; oder zur Herstellung eines Kniegelenks für eine Osteosynthese einer intraartikulären Fraktur, einer Osteochondritis dissecans, einer Fraktur der proximalen Tibia oder zur Osteosynthese in der Osteotomie.

18. Formteil nach Anspruch 11, dadurch **gekennzeichnet,** daß das Formteil einen Durchmesser von 2,0 bis 4,0 mm hat.

19. Verwendung eines Formteils nach Anspruch 18 zur Herstellung eines Mittels, das in einem Knöchelgelenk zur Osteosynthese von Osteochondritis dissecans und einer osteochondralen Fraktur verwendet wird.

20. Formteil nach Anspruch 11, dadurch **gekennzeichnet,** daß das Formteil einen Durchmesser von 3,5 bis 4,5 mm hat.

**21.** Verwendung eines Formteils nach Anspruch 20 zur Herstellung eines Kniegelenks zur Osteosynthese einer Fraktur des femoralen Condylus.

**22.** Formteil nach Anspruch 11, dadurch **gekennzeichnet,** daß das Teil einen Durchmesser von 1,5 bis 2,5 mm hat.

**23.** Verwendung eines Formteils nach Anspruch 22 zur Herstellung eines Mittels zur Verwendung in Zehen bei einer Operation eines krummen Hallux oder bei der Osteosynthese einer Fraktur der Zehenphalanx.

**24.** Formteil nach Anspruch 1, dadurch **gekennzeichnet,** daß das Teil ein Formteil die Gestalt einer Mikroschnecke hat.

**25.** Formteil nach Anspruch 24, dadurch **gekennzeichnet,** daß das Teil einen Durchmesser von 1,5 bis 2,5 mm hat.

**26.** Verwendung eines Formteils nach Anspruch 25 zur Herstellung eines Mittels, das in einem Handgelenk und in einer Fingerphalanx zur Osteosynthese einer Fraktur der distalen Speiche und einer Fraktur der Fingerphalanx verwendet wird.

**27.** Formteil nach Anspruch 1, dadurch **gekennzeichnet,** daß das Teil ein gebogenes oder ein gerades stabförmiges Formteil ist.

**28.** Formteil nach Anspruch 27, dadurch **gekennzeichnet,** daß das Teil einen Durchmesser von 2,0 bis 4,0 mm hat.

**29.** Verwendung eines Formteils nach Anspruch 28 zur Herstellung eines Mittels zur Osteosynthese einer Fraktur des Schlüsselbeins oder einer Rippe.

**30.** Formteil nach Anspruch 1, dadurch **gekennzeichnet,** daß das Teil eine Kombination aus einem plattenförmigen Formteil, das mit Löchern durchbohrt ist und das eine Dicke zwischen 1,0 und 2,0 mm hat, und einem schneckenförmigen Formteil, das einen Durchmesser zwischen 2,0 und 2,5 mm hat, zur Fixierung des plattenförmigen Formteil ist.

**31.** Verwendung eines Formteils nach Anspruch 30 zur Herstellung eines Mittels, das in der Oralchirurgie zur Osteosynthese von Frakturen des Schädels, des Jochbeins, des Nasenknochens, des Oberkiefers und des Unterkiefers verwendet wird.

**Revendications**

**1.** Article moulé biodégradable et résorbable à usage chirurgical, lequel comprend un article moulé et étiré en acide polylactique ayant une masse moléculaire moyenne à l'état visqueux comprise entre 200 000 et 500 000, une résistance à la flexion en compression comprise entre 16,0 x $10^2$ et 25,0 x $10^2$ kg/cm², un module de flexion en compression compris entre 5,5 x $10^2$ et 24,0 x $10^2$ kg/mm² et un degré de cristallinité compris entre 10 et 60 %, tel que calculé d'après la densité, l'épaisseur de la partie la plus épaisse dudit article moulé et étiré étant maintenue entre 1,0 mm et 4,5 mm de façon que la résistance mécanique dudit article soit détériorée par dégradation dans un organisme vivant en l'espace d'environ 4 à 16 semaines et qu'il soit résorbé dans l'organisme vivant à au moins 50 % en une durée comprise entre environ 1,5 et 2,0 ans.

**2.** Article moulé selon la revendication 1, caractérisé en ce que ledit article est un article moulé en forme de vis.

**3.** Article moulé selon la revendication 2, caractérisé en ce que ledit article a un diamètre compris entre 2,5 et 3,5 mm.

**4.** Utilisation d'un article moulé selon la revendication 3 pour préparer une articulation scapulo-humérale pour la fixation de la grosse tubérosité, de fragments ostéoclastiques du col chirurgical et de fragments de la cavité glénoïde ;
ou pour préparer un bassin et/ou une articulation coxo-fémorale pour l'ostéosynthèse d'une fracture de l'acétabulum et d'une fracture par luxation de l'ilion, pour la fixation d'un greffon dans une arthroplastie de la hanche et pour l'ostéosynthèse d'une fracture de la tête fémorale ;
ou pour préparer un moyen d'ostéosynthèse d'une fracture intra-articulaire, d'une ostéochondrite disséquante, d'une fracture du tibia proximal ou d'ostéosynthèse dans l'ostéotomie ;
ou pour préparer un moyen d'ostéosynthèse d'une fracture de la malléole interne, externe ou postérieure d'une articulation tibio-tarsienne.

**5.** Article moulé selon la revendication 2, caractérisé en ce que ledit article a un diamètre compris entre 2,0 et 3,0 mm.

**6.** Utilisation d'un article moulé selon la revendication 5 pour préparer une articulation du coude pour l'ostéosynthèse d'une fracture de l'épicondyle ou de l'épitrochlée.

**7.** Article moulé selon la revendication 2, caractérisé en ce que ledit article a un diamètre compris entre 3,5 et 4,5 mm.

**8.** Utilisation d'un article moulé selon la revendication 7 pour préparer une articulation de genou pour l'ostéosynthèse d'une fracture du condyle fémoral ;
ou pour préparer un moyen d'ostéosynthèse d'une fracture de l'astragale dans une articulation tibio-tarsienne ;
ou pour préparer un moyen à utiliser dans la diaphyse d'un os long pour la fixation d'un greffon osseux.

**9.** Article moulé selon la revendication 2, caractérisé en ce que ledit article a un diamètre compris entre 2,0 et 3,5 mm.

**10.** Utilisation d'un article moulé selon la revendication 9 pour préparer un moyen à utiliser dans une colonne vertébrale cervicale pour décompression antérieure et arthrodèse et pour la fixation d'un greffon osseux.

**11.** Article moulé selon la revendication 1, caractérisé en ce que ledit article est un article moulé en forme de clou.

**12.** Article moulé selon la revendication 11, caractérisé en ce que ledit article a un diamètre compris entre 1,0 et 2,0 mm.

**13.** Utilisation d'un article moulé selon la revendication 12 pour préparer un moyen à utiliser dans des carpes et des métacarpiens pour l'ostéosynthèse d'une fracture de phalange de doigt en divers sites et d'une fracture intra-articulaire, d'une fracture du radius distal.

**14.** Article moulé selon la revendication 11, caractérisé en ce que ledit article a un diamètre compris entre 1,0 et 2,5 mm.

**15.** Utilisation d'un article moulé selon la revendication 14 pour préparer une articulation de coude pour une fracture ostéo-chondrale et la fixation d'un greffon osseux pour soigner l'épicondylite des joueurs de base-ball.

**16.** Article moulé selon la revendication 11, caractérisé en ce que ledit article a un diamètre compris entre 2,5 et 3,5 mm.

**17.** Utilisation d'un article moulé selon la revendication 16 pour préparer un bassin et/ou un articulation coxo-fémorale pour l'ostéosynthèse d'une fracture de l'acétabulum et d'une fracture par luxation de l'ilion, pour la fixation d'un greffon dans une arthroplastie de la hanche et pour l'ostéosynthèse d'une fracture de la tête fémorale ;
ou pour préparer une articulation de genou pour l'ostéosynthèse d'une fracture intra-articulaire, d'une ostéochondrite disséquante, d'une fracture du tibia proximal ou pour l'ostéosynthèse dans l'ostéotomie.

**18.** Article moulé selon la revendication 11, caractérisé en ce que ledit article a un diamètre compris entre 2,0 et 4,0 mm.

**19.** Utilisation d'un article moulé selon la revendication 18 pour préparer un moyen utilisé dans une articulation tibio-tarsienne pour l'ostéosynthèse d'une ostéochondrite disséquante et d'une fracture ostéo-chondrale.

**20.** Article moulé selon la revendication 11, caractérisé en ce que ledit article a un diamètre compris entre 3,5 et 4,5 mm.

**21.** Utilisation d'un article moulé selon la revendication 20, pour préparer une articulation de genou pour l'ostéosynthèse d'une fracture du condyle fémoral.

**22.** Article moulé selon la revendication 11, caractérisé en ce que ledit article a un diamètre compris entre 1,5 et 2,5 mm.

**23.** Utilisation d'un article moulé selon la revendication 22 pour préparer un moyen à utiliser dans des orteils au cours d'une intervention pour hallux valgus ou dans une ostéosynthèse d'une fracture de phalange d'orteil.

**24.** Article moulé selon la revendication 1, caractérisé en ce que ledit article est un article moulé en forme de microvis.

**25.** Article moulé selon la revendication 24, caractérisé en ce que ledit article a un diamètre compris entre 1,5 et 2,5 mm.

**26.** Utilisation d'un article moulé selon la revendication 25 pour préparer un moyen utilisé dans une articulation radio-carpienne et une phalange de doigt pour l'ostéosynthèse d'une fracture du radius distal et d'une fracture de phalange de doigt.

27. Article moulé selon la revendication 1, caractérisé en ce que ledit article est un article moulé en forme de tige incurvée ou droite.

28. Article moulé selon la revendication 27, caractérisé en ce que ledit article a un diamètre compris entre 2,0 et 4,0 mm.

29. Utilisation d'un article moulé selon la revendication 28 pour préparer un moyen pour l'ostéosynthèse d'une fracture d'une clavicule ou d'une côte.

30. Article moulé selon la revendication 1, caractérisé en ce que ledit article est une combinaison d'un article moulé en forme de plaque, percé de trous, d'épaisseur comprise entre 1,0 et 2,0 mm, et d'un article moulé en forme de vis, de diamètre compris entre 2,0 et 2,5 mm, destiné à fixer ledit article moulé en forme de plaque.

31. Utilisation d'un article moulé selon la revendication 30 pour préparer un moyen utilisé en chirurgie buccodentaire et maxillaire pour l'ostéosynthèse de fractures crâniennes, de fractures d'os malaire, d'os propre du nez, de maxillaire supérieur et de maxillaire inférieur.

# Fig.1

(a)

(b)

# Fig.2

(a)  (b)

# Fig.3

# Fig.4

## (a)

## (b)

# Fig.5

# Fig. 6

(a)

(b)

# Fig. 7

100

4

Fig.8

Fig.9

φ 3.2 mm
φ 4.0 mm
φ 5.0 mm

Bending Strength

kg/cm²
2500
2000
1000
0

4   8   12   16   20   25   Week

Fig.10

Fig.11

Change of Amount in Vivo ( φ3.2 mm rod )